# EUROPÄISCHE PATENTSCHRIFT

(11) **EP 1 293 164 B1**
(45) Veröffentlichungstag und Bekanntmachung des Hinweises auf die Patenterteilung: **05.08.2009**
(21) Anmeldenummer: 02020381.6
(22) Anmeldetag: 12.09.2002
(51) Int. Cl.: A61B 5/20, A61B 10/00, G01N 31/22, G01N 33/493

(54) **Vorrichtung zum Nachweis von Partikeln im Urin**
Device for determining particles in urine
Appareil pour determiner des particules dans l'urine

(30) Priorität: 14.09.2001 DE 10145424
(43) Veröffentlichungstag der Anmeldung: 19.03.2003
(73) Patentinhaber: Sarstedt AG & Co., 51588 Nümbrecht (DE)
(72) Erfinder: Sarstedt, Walter, 51588 Nümbrecht (DE); Färber, Horst, 51588 Nümbrecht (DE); Flach, Dagmar, Dr., 51643 Gummersbach (DE); Gross, Petra, Dr., 51674 Wiehl (DE)
(74) Vertreter: Grosse, Wolf-Dietrich Rüdiger

(56) Entgegenhaltungen:
- US-A- 4 473 530
- US-A- 4 973 450
- PATENT ABSTRACTS OF JAPAN vol. 1999, no. 08, 30. Juni 1999 (1999-06-30) & JP 11 064183 A (CHO BOKEI), 5. März 1999 (1999-03-05)

## Beschreibung

Die Erfindung betrifft eine Vorrichtung zur Durchführung eines Tests zum Nachweis von Partikeln im Urin, insbesondere zur Durchführung eines Tests zur Bestimmung der Anfälligkeit einer Testperson für Nierensteine, wobei Urin von der Testperson abgegeben und aufgefangen und anschließend der Nachweistest durchgeführt wird, wobei der Urin nach einer Standzeit aus dem Testgefäß ausgegossen wird bei Zurückbleiben von Partikeln im Testgefäß mit anschließender Erkennung des Testergebnisses durch ein Farbreagenz zur optischen Erkennung

Ein solches Testgefäß, ausgeführt als Zentrifugenröhre, ist durch die JP 11 064 183 A bekannt geworden. In dieses Zentrifugenröhrchen muß der von einer Person zuvor in einen großvolumigen Auffangbehälter abgegebene Urin umgefüllt werden. Vor dem Zentrifugieren, nachdem sich eine für den Test kleine Urinmenge im geschlossenen unteren, konisch ausgebildeten Ende des Zentrifugierröhrchens befindet, wird die darüber stehende Urinmenge aus dem offenen, oberen Röhrchenende ausgegossen Das Röhrchen wird dann in eine Zentrifuge eingestellt.

Aus der EP 0 889 326 A1 ist ein Baukastensystem zur Durchführung eines Nierensteintestes bekannt. Um die Urineigenschaften eines Patienten hinsichtlich der Anfälligkeit für Nierensteine zu testen, wird der Urin dahingehend geprüft, ob kalziumreiche Salze bzw. Partikel ausscheiden. Es ist bekannt, folgende wesentliche Schritte hintereinander durchzuführen:
Gießen von 40ml von gerade abgegebenem Urin einer Testperson in eine Petrischale bzw. in eine mittig zu der Petrischale angeordneten Schale, die ein Reaktionssubstrat enthält,
Durchführen einer Standzeit (6 bis 24 Stunden) zum Ausscheiden von Kalzium-Partikeln;
Ausschütten des Petrischaleninhalts sowie anschließendes Reinigen mit destilliertem Wasser;
Hinzufügen von 400µl einer verdünnten wäßrigen HCI-Lösung auf das Reaktionssubstrat;
Hinzufügen von 150µl einer wäßrigen Natriumacetat-Lösung (5%, w/v) und Hinzufügen von 150µl der Indikator-Lösung Arsenazo III (0,1%, w/v) sowie Vermischen für 15 Sekunden,
anschließende optische Erkennung des Testergebnisses durch eine pinke Farbe der Lösung bei einer nicht anfälligen Testperson sowie durch eine blaue Farbe der Lösung bei einer anfälligen Testperson, wobei die blaue Farbe eine Schattierung von violett und blau aufweisen kann. Im Grenzbereich zwischen einem positiven und negativen Befund weist die Lösung eine pinke Farbe mit einem schwachen bläulichen Ton auf.

Es wird als wesentlich hervorgehoben, daß der Test mit gerade abgegebenem und somit noch warmem Urin durchzuführen ist, um zu vermeiden, daß das Testergebnis durch verfrühte Ausscheidungen aufgrund von Abkühlung verfälscht wird.

Der Bausatz zur Durchführung des Testes setzt sich aus einer Petrischale mit einem Deckel zusammen sowie einer kleineren Mittelschale zur Aufnahme des Reaktionssubstrates sowie der benötigten Reagenzien.

Dieses Verfahren bzw. System weist die folgenden Nachteile auf.

Da der Urin noch warm auf die Petrischale gegeben werden muß, ist ein separates Auffangbehältnis zum Abgeben des Urins nötig. Da der Test auch als Vorabtest von den Testpersonen zu Hause durchgeführt werden soll, muß die Testperson ggfs. durch eine schriftliche Gebrauchsanweisung die Anleitung erhalten, daß sie den Urin in einem separaten Behälter aufzufangen hat und unmittelbar danach noch warm auf die Petrischale zu gießen hat. Zudem muß die Testperson oder der Anwender eine definierte Urinmenge, nämlich genau 40ml, in die Petrischale geben, was eine vorherige Abmessung der Urinmenge notwendig macht. Obwohl die Petrischale nach dem bekannten System einen Deckel aufweist, wird sich ein möglicher Transport der Petrischale als außerordentlich schwierig und gerade für ältere Menschen als unpraktikabel erweisen.

Das bekannte Verfahren zum Nachweis von positivem oder negativem Nierensteinverdacht beinhaltet des weiteren eine Reihe von Bearbeitungsschritten, wie Reinigen und Zugabe einer Mehrzahl von Reagenzien, wobei sowohl auf eine genaue Menge an Zugabeflüssigkeiten als auch auf das Einhalten von Zeitangaben für Handlungen, wie zum Beispiel Umrühren, zu achten ist.

Das Reagenz Arsenazo III ist zudem giftig und führt daher zu Entsorgungsproblemen beim Anwender.

Das Testergebnis wird an der Farbe der Lösung abgelesen, wobei gerade der Grenzbereich (pinke Farbe mit schwachem bläulichen Ton) zwischen einem eindeutigen positiven oder negativen Ergebnis sehr schlecht zu erkennen ist, was dadurch beeinflußt wird, daß das positive Ergebnis eine blaue Farbe von violett bis blau hervorbringt.

Der Erfindung liegt ausgehend von der JP 11 064 183 A die Aufgabe zugrunde, eine Vorrichtung zur Durchführung eines Tests zum Nachweis von Partikeln im Urin bereitzustellen, die die Einstellung einer definierten Menge Test-Urin im Testgefäß und das Verschließen des Testgefäßes erlaubt, ohne dass beim Verschließen des Testgefäßes Urin austreten kann. Außerdem soll eine Testperson den Urin ohne Umfüllung direkt in ein Testgefäß geben können.

Diese Aufgabe wird durch die Merkmale des Anspruchs 1 gelöst. Im einzelnen wird die Aufgabe dadurch gelöst, daß die Vorrichtung einen Auffangbehälter aufweist, in den von der Testperson unmittelbar Urin abgegeben wird und der gleichzeitig auch Testgefäß ist. Hierbei ist das Testgefäß säulenförmig bzw. rohrförmig, sich zum Boden hin verjüngend, ausgeformt und geht nach oben hin in einen - vorzugsweise an das Testgefäß einstückig angeformten - Überlaufbereich des Auffangbehälters über, der vorzugsweise eine Soll-Füllstandmarkierung zur Orientierung für die Testperson aufweist. Zudem weist die Vorrichtung ein Verschlußelement auf, das in den Auffangbehälter bzw. in den Überlaufbereich eintaucht und unter Verdrängung von zu viel eingegebenem Urin in den Überlaufbereich das Testgefäß zur Einstellung einer definierten Menge Test-Urin derart verschließt, dass durch den Verdrängungseffekt die Urinsäule im Überlaufbereich von einem ersten Füllstand I etwa auf Höhe der vorzugsweise vorgesehenen Soll-Füllstandsmarkierung bis zu einem zweiten Füllstand II des Überlaufbereichs bei verschlossenem Auffangbehälter steigt.

Vorzugsweise weist die Vorrichtung Standmittel auf zum selbständigen Aufstellen des Auffangbehälters mit vertikaler Ausrichtung zur Durchführung der Standzeit.

Die vorgeschlagene Vorrichtung zur Durchführung des Tests bietet folgende Vorteile und weist folgende bevorzugte Weiterentwicklungen auf:

Die Testperson bzw. der Patient kann den Urin direkt in das Testgefäß geben. Damit ist sichergestellt, daß der Urin - was für den Test unbedingt notwendig ist - warm in das Gefäß gelangt und nicht durch irgendwelche notwendigen Handhabungen, wie Umfüllungen, abkühlt.

Zudem kann die für den Test notwendige definierte Menge an Urin erhalten werden, indem sich die Testperson einerseits an der Füllstandmarkierung orientieren kann und andererseits der Überlaufbereich sowie das Verschlußelement durch den Verdrängungseffekt garantieren, daß nach ordnungsgemäßem Verschluß sich innerhalb des Testgefäßes die geforderte Menge an Urin befindet. D.h., das Verschlußelement, bei dem es sich vorzugsweise um einen Schraubverschluß handelt, greift in den vorzugsweise trichterförmigen Überlaufbereich ein, der das Testgefäß mit einem definierten Volumen am Ende des Schraubvorgangs abdichtet.

Das Testgefäß - als unterer Teil des Auffangbehälters - ist erfindungsgemäß säulenförmig bzw. rohrförmig, sich zum Boden hin verjüngend, ausgeformt und ergibt damit eine Zapfenform. Durch diese Form wird gewährleistet, daß sich die aus der übersättigten Urinlösung ausgeschiedenen Partikel konzentriert auf einen begrenzten Ort absetzen können.

Als Nachweisreagenz wird ein Farbreagenz gewählt, das ungefährlich und ungiftig ist, beispielsweise aus der Gruppe der Calciumindikatoren.

Derartige Farbreagenzien haben den Vorteil, daß sie eine eindeutigere Erkennung des Testergebnisses als das Farbreagenz aus der EP 0 889 326 A1 zulassen, indem die Farben klar zuzuordnen sind. So zeigen negative Ergebnisse ein Erdbeerrot, während positive Befunde ein Dunkelrot zeigen. Sollte der Beurteiler Zweifel an der Zuordnung der Farbe haben, so kann ein zweiter Tropfen Farbreagenz dazugegeben werden. Die Farbe entwickelt sich dann je nach Beschaffenheit der Urinprobe entweder deutlich mehr ins Erdbeerrot oder ins Dunkelrot.

Um dem Beurteiler das Ablesen des Ergebnisses mit Hilfe von unterschiedlichen Farben zu erleichtern, weist der sich verjüngende Boden des säulenförmigen Testgefäßes zusätzlich einen küvettenartigen Fortsatz auf, in den das Farbreagenz bevorzugt gegeben wird. Die vorgeschlagene Geometrie trägt zur guten optischen Unterscheidung verschiedener Farbgebungen bei. Zudem ist im Vergleich zum Stand der Technik nach der EP 0 889 326 A1 nur die Zugabe von einem Reagenz notwendig.

Weitere Einzelheiten und Vorteile der Erfindung ergeben sich aus der nachfolgenden Beschreibung, in der eine in den Figuren dargestellte Ausführungsform der Erfindung näher erläutert wird. Es zeigen:
- Fig. 1: eine Schnittansicht der erfindungsgemäß vorgeschlagenen Vorrichtung zur Durchführung eines Nierensteintests;
- Fig. 2: eine Schnittansicht der Vorrichtung nach Fig. 1, um 90°C gedreht;
- Fig. 3: den Querschnitt A-A der Fig. 1.

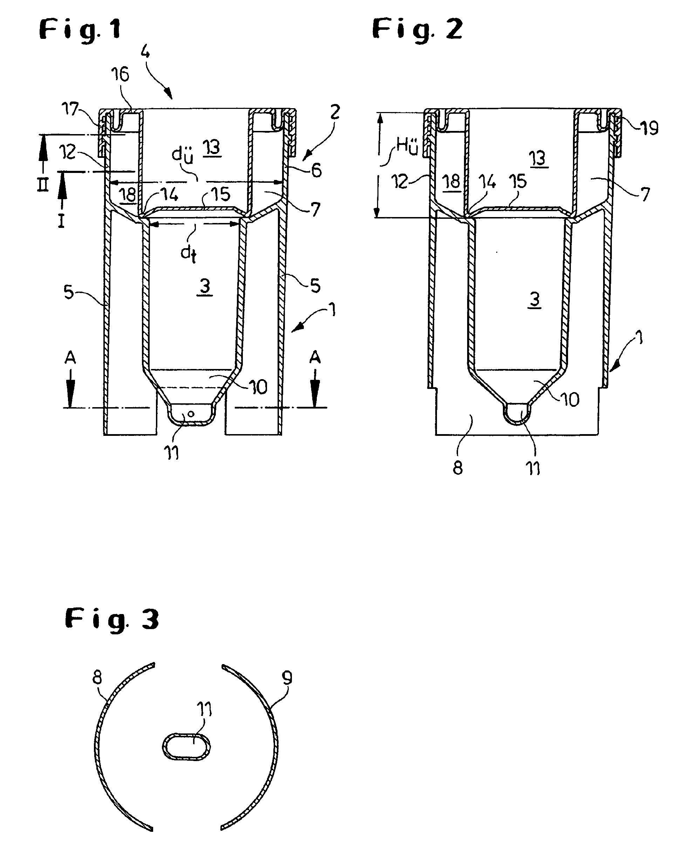

Die Gesamtvorrichtung 1 zur Durchführung des Nierensteintests setzt sich aus einem Auffangbehälter 2, der gleichzeitig auch Testgefäß 3 ist, einem Schraubverschluß als Verschlußelement 4 sowie Standmitteln 5 für den Auffangbehälter 2 zusammen, die einstückig aus der Wandung 6 eines Überlaufbereichs 7 nach unten fortgezogen sind und eine aus zwei Halbschalen 8, 9 gebildete sichere Standfläche erzeugen (vgl. Fig. 3).

Der Auffangbehälter 2 selbst setzt sich aus dem unteren Testgefäß 3 und dem sich einstückig nach oben daran anschließenden trichterförmigen Überlaufbereich 7 zusammen. Der Auffangbehälter 2 kann mit den Standmitteln 5 bzw. -flächen einstückig aus Kunststoff spritzgegossen werden.

Das Testgefäß 3 ist von einer länglichen zapfenartigen Form, die nach unten hin konisch zuläuft, wobei die Verjüngung 10 in einen küvettenartigen Fortsatz 11 einläuft, der aufgrund seiner Geometrie zum einen die konzentrierte Sedimentation der Partikel unterstützt sowie zum anderen zur besseren optischen Unterscheidung verschiedener Farbgebungen des Farbreagenz beiträgt. Außerdem wird die Vermischung von Farbreagenz und eventuell vorhandenen, nachzuweisenden Partikeln erleichtert.

Das Testgefäß 3 weist je nach Testart ein sich gemäß der Urinmenge entsprechendes Volumen, hier beispielsweise 24ml, auf.

Nach oben schließt sich an das Testgefäß 3 der trichterförmige Überlaufbereich 7 an, dessen Durchmesser d_{ü} stets größer ist als der Durchmesser d_{T} des Testgefäßes am oben Rand. An diesem Überlaufbereich 7 ist von außen sichtbar eine Füllstandmarkierung 12 angebracht, an der sich die Testperson beim Abgeben des Urins orientieren kann. Zum Verschließen des Auffangbehälters 2 wird das Verschlußelement 4, das sich zusammensetzt aus einem sich über die Höhe H_{ü} des Überlaufbereichs 7 erstreckenden Verdrängungskörper 13 mit seitlichen Vorsprüngen 14 und einer etwas zurückgenommenen Kontaktfläche 15 sowie einem von dem Verdrängungskörper 13 radial - den Überlaufbereich 7 mittels eines Klammerelementes 16 überbrückenden - beabstandeten Schraubgewinde 17, in den mit Urin gefüllten Auffangbehälter 2 eingedreht. Ein über die gewünschte Testmenge Vₛₒₗₗ im Testgefäß hinausgehender Teil des Urins wird durch das Eintauchen des Verdrängungskörpers 13 beim Eindrehen verdrängt und die exakte Urinmenge Vₛₒₗₗ eingestellt, ohne daß die Testperson hierfür von sich aus hätte eine Messung vornehmen müssen.

Durch den Verdrängungseffekt steigt die Urinsäule 18 im Überlaufbereich 7 von einem ersten Füllstand I etwa auf Höhe der Füllstandmarkierung 12 bis zu einem Füllstand II bei verschlossenem Behälter. Die Füllstandmarkierung 12 hat hierbei zwei wesentliche Vorteile, zum einen wird hierdurch erreicht, daß genügend Urin im Auffangbehälter gesammelt wird, um auf jeden Fall die notwendige Testmenge zu gewährleisten, zum anderen ist die verdrängte Urinmenge nicht so groß, daß Urin beim Steigen der Urinsäule aus dem Überlaufbereich austreten kann. Auf diese Weise wird die vorgeschlagene Testvorrichtung einfach händelbar, auch für einen Selbsttest Zuhause.

Die Gegenüberstellung der beiden Schnitte der Fig. 1 und Fig. 2, wobei gleiche Teile mit entsprechenden Bezugszeichen versehen sind, verdeutlicht die Geometrie des küvettenartigen Fortsatzes 11, der in etwa eine Kanalform aufweist.

Die Form der Standmittel 5 wird durch die Fig. 3 verdeutlicht. Die Standflächen werden durch zwei Halbschalen 8,9 gebildet, die im oberen Teil des Behälters die Überlaufbereichswände 6 ausbilden und mit einem Außengewinde 19 zum Eindrehen des Schraubverschlusses nach oben hin abschließen. Diese Standfüße gewährleisten, daß der Auffangbehälter 2 zur Überbrückung einer Standzeit sicher aufgestellt werden kann mit vertikaler Ausrichtung des Testgefäßes 3, wobei durch den Abstand der Halbschalen 8,9 ein Sichtfenster für das Testgefäß 3, insbesondere dessen Bodenbereich 10 mit küvettenartigem Fortsatz 11, verbleibt.

Der Nierensteintest soll als Baukastensystem zur Anwendung kommen, das die Vorrichtung in Form des Auffangbehälters mit integriertem Testgefäß sowie eine mit dem Farbreagenz gefüllte - beispielsweise - Einweg-Pipette umfaßt, wobei das Volumen des Farbreagenz etwa im Bereich von 300µl liegen soll, das in Einzelschritten von etwa 100µl in das Testgefäß einpipettiert wird. Sollte beim ersten Nachweisversuch keine eindeutige Erkennung möglich sein, so können in einem zweiten und, wenn erforderlich, in einem dritten Schritt etwa 100µl Farbreagenz für eine Intensivierung der Farben in das Testgefäß einpipettiert werden.

Die vorgeschlagene Vorrichtung sowie das Verfahren sind nicht auf einen Nierensteintest beschränkt, sondern können auch Anwendung bei anderen Urintests finden. Insbesondere soll der Nierensteintest Anwendung finden als Selbsttest für Zuhause, aber auch als Vorabtest in Apotheken, Arztpraxen oder ggfs. in Krankenhäusern.

## Patentansprüche

1. Vorrichtung (1) zur Durchführung eines Tests zum Nachweis von Partikeln im Urin, insbesondere zur Durchführung eines Tests zur Bestimmung der Anfälligkeit einer Testperson für Nierensteine, wobei Urin von der Testperson abgegeben und aufgefangen und anschließend der Nachweistest durchgeführt wird, wobei der Urin nach einer Standzeit aus dem Testgefäß ausgegossen wird bei Zurückbleiben von Partikeln im Testgefäß mit anschließender Erkennung des Testergebnisses durch ein Farbreagenz zur optischen Erkennung,
**gekennzeichnet durch**
- einen Auffangbehälter (2), in den von der Testperson unmittelbar Urin abgegeben wird, der gleichzeitig auch Testgefäß (3) ist,
wobei das Testgefäß (3) säulenförmig, sich zum Boden hin verjüngend, ausgeformt ist und nach oben hin in einen Überlaufbereich (7) des Auffangbehälters (2) übergeht, sowie
- ein Verschlußelement (4), das in den Überlaufbereich (7) eintaucht und unter Verdrängung von zu viel eingegebenem Urin in den Überlaufbereich das Testgefäß (3) zur Einstellung einer definierten Menge Test-Urin verschließt, wobei **durch** den Verdrängungseffekt die Urinsäule (18) im Überlaufbereich (7) von einem ersten Füllstand I bis zu einem zweiten Füllstand II des Überlaufbereichs bei verschlossenem Auffangbehälter (2) steigt.

## Claims

1. Apparatus (1) for carrying out a test for the detection of particles in urine, in particular for carrying out a test to determine the susceptibility of a test subject to kidney stones, wherein urine is deposited by the test subject and collected and then the detection test is carried out, wherein the urine after a stand time is poured out of the test vessel, with particles remaining behind in the test vessel, with subsequent detection of the test result by a dye reagent for optical detection,
**characterized by**
- a collecting container (2) into which urine is directly deposited by the test subject and which is, at the same time, also the test vessel (3),
the test vessel (3) being formed in a column shape, tapering toward the bottom and continuing upwards into an overflow region (7) of the collecting container (2), and
- a closure element (4) which dips into the overflow region (7) and, with displacement into the overflow region of excess delivered urine, closes the test vessel (3) to establish a defined quantity of test urine, wherein through the displacement effect the urine column (18) in the overflow region (7) rises from a first filling level I up to a second filling level II of the overflow region with the collecting container (2) closed.

## Revendications

1. Dispositif (1) de réalisation d'un test pour la mise en évidence de particules dans l'urine, en particulier pour la réalisation d'un test de détermination de la sensibilité d'une personne testée aux calculs rénaux, de l'urine étant prélevée et collectée auprès de la personne testée puis le test de mise en évidence étant réalisé, l'urine étant versée du récipient de test après un temps de pose si des particules subsistent dans le récipient de test, avec détermination consécutive du résultat du test au moyen d'un réactif coloré permettant la reconnaissance optique,
**caractérisé par**
- un récipient collecteur (2) dans lequel l'urine est émise directement par la personne testée et qui sert en même temps de récipient de test (3),
le récipient de test (3) étant conformé en forme de colonne se rétrécissant vers le fond et transitant vers le haut dans une zone de débordement (7) du récipient collecteur (2), et
- un élément de fermeture (4) plongeant dans la zone de débordement (7) et fermant le récipient de test (3) sous le refoulement de l'urine émise en excédent dans la zone de débordement pour régler une quantité définie d'urine de test, la colonne d'urine (18) montant, sous l'effet de refoulement, dans la zone de débordement (7) d'un premier niveau de remplissage I à un deuxième niveau de remplissage II de la zone de débordement lorsque le récipient collecteur (2) est fermé.
